Europäisches Patentamt

European Patent Office

Office européen des brevets

(11) Publication number: **0 444 638 A2**

# EUROPEAN PATENT APPLICATION

(21) Application number: 91102935.3

(22) Date of filing: 27.02.91

(51) Int. Cl.5: **C12N 15/12**, C12N 7/01, C12N 5/16, A61K 37/02

(30) Priority: **27.02.90 IT 4153890**

(43) Date of publication of application: **04.09.91 Bulletin 91/36**

(84) Designated Contracting States: **AT BE CH DE DK ES FR GB GR IT LI LU NL SE**

(71) Applicant: **FIDIA S.p.A. Via Ponte della Fabbrica 3-A I-35031 Abano Terme (Padova)(IT)**

(72) Inventor: **Della Valle, Francesco Via Cerato 14 Padova(IT)**
Inventor: **Callegaro, Lanfranco Via Bravi 35 IT-35020 Ponte di Branta Padova(IT)**
Inventor: **Negro, Alessandro Via Umago 5 Padova(IT)**

(74) Representative: **Vossius & Partner Siebertstrasse 4 P.O. Box 86 07 67 W-8000 München 86(DE)**

(54) Process for the expression of human nerve growth factor in arthropoda frugiperda cells by infection with recombinant baculovirus.

(57) A process is disclosed for obtaining a polypeptide called human nerve growth factor (hβNGF) from cells which have been infected or transformed with a recombinant Baculovirus, and more precisely, a process to obtain infected insect cells which produce the mature, biologically active, human form of this factor, by recombinant DNA technology starting with genetic constructs to be inserted into Arthropoda Frugiperda cell lines.

EP 0 444 638 A2

The present invention concerns a process to obtain a polypeptide called human nerve growth factor (hβNGF) from cells which have been infected or transformed with a recombinant Baculovirus, and more precisely, a process to obtain infected insect cells which produce the mature, biologically active, human form of this factor, by recombinant DNA technology starting with genetic constructs to be inserted into Arthropoda Frugiperda cell lines.

A. Nerve Growth Factor (NGF)

Nerve Growth Factor (NGF) was first discovered in mouse sarcoma tumours (Levi-Montalcini, R. et al., J. Exp. Zool. 116:321, 1951) and was then purified to homogeneity from male mouse submaxillary glands (Varon, S. et al., Biochemistry 6:2202, 1967) and from snake venom (Angeletti, R. H., Proc. Natl. Acad., Sci. USA 65:668, 1970). Many other relatively rich sources of NGF have been reported, including guinea pig prostate (Harper, G. P. et al., Nature 279:160, 1979) and human placenta (Goldstein, L. D. et al., Neurochem. Res. 3:175, 1978, Walker, P. et al., Life Science 26:195, 1980, Fidia patent 47745A88). Small quantities of NGF have been found in other tissues such as the mammalian central nervous system (Varon, S., Discussions in Neuroscience, Vol. II, No. 3, 1985; Hefti, F., et al., Neuroscience 14:55, 1985). The physiological correlation between these potential sources of NGF and its apparent action sites is none too clear, but it is generally supposed that NGF is secreted from the various peripheral tissues which require innervation by those cells which respond to NGF.

NGF from mouse submaxillary glands is the kind most widely used for studies on the activity of NGF in vitro and in vivo. Its range of biological activity has been determined in vitro both on primary neuronal cells and on cloned cell lines. Among the primary neuronal cells which have responded to NGF in vitro are fetal sensory neurons (fetal days 8-12) from spinal ganglion root, autonomic, noradrenergic, fetal neurons from sympathetic ganglia, cholinergic fetal neurons from the septum and chromaffin adrenal cells during development. While the sensory and sympathetic neurons depend on NGF for survival and development, cholinergic neurons do not appear to need NGF for survival but only for their differentiation, that is, for the expression of characteristic phenotype features linked with the neurotransmitter. The addition of NGF to chromaffin adrenal cells (derived from the neuronal crest) during the first stage of their development causes the expression of neuronal phenotypes. Among the cell lines which respond, as described in the literature, to NGF in vitro, included are chromaffin adrenal cells derived from tumours of the neuronal crest, called pheochromocytoma (PC12) cells, and human neuroblastoma cells. After treatment with βNGF these cells behave differently, passing from a highly proliferative phase to a postmitotic condition.

The nerve growth factor obtained from mouse submaxillary glands has been better characterized than other kinds, also from a chemical and immunochemical point of view. NGF from murine glands acts as a 7S-type protein complex (molecular weight about 140,000 daltons) composed of three different subunits (α, β, γ) which coordinate a Zn+ atom. The most interesting part of the 7S molecule, relative to its biological activity, is constituted by two polypeptide chains, each with a molecular weight of 13,250 and formed by 118 amino acids. Each chain or monomer has three disulphide bridges, which form covalent links between two cysteine amino acid residues, conferring strong stability to the three-dimensional structure of the protein. The two monomers of NGF, joined together by weak bonds, form a dimer with a molecular weight of 26,500. It has been demonstrated that the biological activity is associated with the dimer known as 2.5S, or more conventionally β subunit. Whether or not such activity is present also in the monomer is not known. The present application refers to the biologically active portion of human NGF, whatever type of abbreviation is used, such as hNGF or hβNGF or 2.5 NGF or 2.5 hNGF. Genetic engineering techniques have made it possible to identify the gene which encodes the molecule of β subunit of NGF (βNGF) (Scott, J. et al, Nature 302:538, 1983; Ullrich, A. et al, Nature 303:821, 1983). The human gene which encodes the molecule is localized in the short arm of the chromosome I and encodes by the synthesis of a much larger molecule than that with a molecular weight of 26,500 which constitutes the biologically active molecule. The gene therefore sends instructions for the synthesis of a larger NGF or pro-NGF precursor. It has also been demonstrated that the gene of the β subunit of NGF is highly conserved in different species, from birds to humans (Meier, R. et al, EMBO J. 5:1489, 1986). The nucleotide sequence of βNGF of murine, human, bovine and chick origin allows us to compare the conserved and non-conserved sites of these molecules and their relationship with the biological activity and antigenicity. The overall conservation of βNGF during evolution is surprisingly high. Of the 118 amino acids of the mature form of βNGF purified from male mouse submaxillary glands, only 16 amino acids have changed in bovine βNGF, 19 in that of chick and 11 in the human variety, while only 6 amino acids differ between bovine and human βNGF. All the cysteine residues have been strictly conserved in all species. The reduction of the three S-S bridges of βNGF causes a complete loss of its biological activity. The apparent discrepancy between the high level of overall

2

EP 0 444 638 A2

conservation of the amino acid sequence and a low level of cross-reactivity of an immunochemical kind is due to the fact that the changes in amino acids from species to species are situated in specific "clusters". By using hydropathic tracers it is possible to demonstrate that these changes happen almost exclusively in the hydrophilic sites considered to be potential antigenic determinants. Only one hydrophilic region has proved to be perfectly conserved in the NGF molecules of all the species studied so far.

B. Recombinant DNA Technology

Recombinant DNA technology makes it possible to construct series of vectors able to express large quantities of desired proteins. This new technology allows molecular biologists to assemble DNA sequences to create hybrid molecules capable of producing the desired protein. The method is based on various reactions such as cutting with restriction enzymes, joining the fragments thus obtained with ligase, chemical synthesis of oligonucleotides to be assembled and other methodologies devised in the various laboratories working in the field (Maniatis, T. et al, Molecular Cloning. A Laboratory Manual. Cold Spring Harbor Laboratory, Cold Spring Laboratory NY, 1982). To obtain high levels of expression, the DNA elements to be assembled must present certain essential information, for example, a replication origin, a selection for antibiotics, an expression promoter, transcription activators of the gene of interest and other characteristics known to experts in the art. By suitably combining these elements a vector is obtained, if the gene in question is inserted naturally with respect to the regulatory sequences of transcription and transduction and the resulting plasmid is called an expression plasmid. The expression plasmid or vector is thus able to express the protein in host cells. The protein can then be obtained by purification. The elements (promoters) which naturally control the expression of many genes such as the growth factor, are not very strong in their expression and are activated only under suitable natural conditions which are often unknown.

For this reason, promotors with known activity are used, such as that of the polyhedrin of the Baculovirus, or other promoter gene sequences. The elements used for high levels of expression are therefore a combination of DNA of various origin (eukaryotic, bacterial, viral, etc.) resulting in the different gene portions joining together to form a hybrid. The transcription and transduction activities of a gene depend on there being suitable distances between the regulatory and encoding sequences.

This said, one of the best ways for the regulatory sequences to work well is for the gene to be introduced into the same position as that occupied by the natural gene. One system used is that in which the regulatory sequences also include some amino acids of the encoding sequences. Union with the introduced gene gives rise to a fused protein. If, on the other hand, the fused portion is then removed, it is possible to obtain higher biological values. If the technique employing fusion proteins is not used, conventional technologies for obtaining genes situated in the close vicinity of the regulatory sequences depend on there being suitable restriction sites which allow for cloning. If there are no compatible sites nearby, but only different sites, it is possible to obtain union of the segments by synthesis of an oligonucleotide or linker containing the desired restriction site. If there are no restriction sites in the vicinity to allow the use of linkers, then the technique of deleting the DNA with Bal 31 or S1 can be used. This possibility does not allow precise deletion and different clones must be checked each time by sequencing to see which is the most suitable. These methods are very limiting for the molecular biologist and consequently alternative strategies must be devised in view of the new technologies now available, such as the Polymerase Chain Reaction (PCR) (Saiki et al, Science 239:487, 1988; Scharf, S.J., Science 233: 1076, 1986). By this technique it is possible to amplify a gene segment up to $10^6$ times. The principle is based on the use of two oligonucleotides which can each be paired onto one of the DNA strands to be amplified. The distance between the two oligonucleotides, with respect to the gene sequence under examination, gives the dimensions of the molecule to be produced. These two oligonucleotides are so constructed that within the sequence of each there is a restriction site which allows their subsequent cloning. This restriction site is either naturally present or is constructed ad hoc by degenerating the minimum number of nucleotides. This approach, which can be defined as site-directed mutagenesis, allows restriction sites to be constructed in those positions decided on by the molecular biologist. The construction of sites which are compatible with other gene segments makes not only for easy cloning, but also, and above all, makes it possible to join different gene segments as desired. This technique can be described as cloning by direct mutagenesis. In practice, it is possible, by recombinant DNA technology, to express complete heterologous polypeptides by direct expression, or alternatively, to express the heterologous polypeptide fused to a portion of the amino acid sequence of an analogous polypeptide. Generally speaking, products thus obtained are not biologically active (British Patent Application Publ. N. 2007676A; Wenzel, American Scientist 68, 664, 1980).

The ability to isolate the human gene of nerve growth factor β subunit opens up an important new possibility. It is possible to produce a sufficient quantity of this rare protein by recombinant DNA

3

technology. Indeed, the nerve growth protein could be accepted for clinical use in treatments for various neurodegenerative diseases. There is documentation on this topic relative to obtaining the NGF subunit $\beta$ by recombinant DNA technology (European Patent No. 0121388; Bruce, G. et al, Neurobiology of Aging 10:89, 1989; Hu, G. et al, Nucleic Acid Research 70:57, 1988; Edwards, R.H., Mol Cell Biol 8:2456, 1988; Emfors, P., Proc Natl Acad Sci 86:4756, 1989; Italian Patent Application No. 48564A89). For production purposes, mammalian cells are preferred to bacteria only in those cases where expression in microbial cells, far less expensive, is not practicable. Indeed, it is far more economical to produce certain proteins in bacterial lines such as E.coli, but generally this host/vector system faithfully reproduces only the linear sequence of the amino acids that make up the protein, obtaining however a kind of in- soluble mass inside the bacterium. Should it be possible to economically prepare a certain product from this material, E.coli could be the preferred system, as in the case of certain smaller molecules, such as interferons and some animal growth proteins where the molecule can be properly folded in vitro. These systems are most profitable in cases generally concerning those proteins with a single disulphide bond and with peptides or proteins which do not require a well-defined structure for their use (as diagnostic antigens or vaccine components).

This invention concerns a process to obtain high levels of expression of the biologically active human nerve growth factor using a system of expression of insect cells/Baculovirus.

Through biotechnology and with various systems it is possible to produce considerable levels of expression of the desired protein. These are not always biologically active however; the present invention takes into consideration both that the levels of expression be high and, above all, that the material be biologically active. In the case of the hNGF molecule, its expression in prokaryotic and eukaryotic systems is known. Regarding its expression in prokaryotic lines (E. coli) it is possible to economically obtain considerable levels of expression, but the polypeptide often contains an initial methionine which cannot be removed. In most cases the material precipitates inside the cells as insoluble particles; their extraction requires coatropic agents and to obtain a biologically active material they must be refolded and checked for dimerization of their molecules. It must be emphasized that the NGF molecule contains three well-structured disulphide bridges and that the molecule's biological activity is due to the association of two molecules forming a dimer and that this retains its biological activity. Mammal cells can be used for the expression of a molecule of human origin but the main drawback to their use is their requirement of large quantities of medium and serum, making them a very expensive source, always an important consideration in industrial processes. Moreover, their levels of expression are generally far lower than those obtainable by a prokaryotic system or by viral expression.

The Baculovirus system overcomes these difficulties because it is more economical in industrial terms, it costs far less in medium than mammal cells, the material keeps its biological activity intact as the cells succeed in forming correct disulphide bridges, and the process of dimer formation to obtain the $\beta$ subunit does not require the refolding of the molecule since this happens naturally. This post-transduction process is important for its biological activity.

Therapeutic proteins, one of which is the nerve growth factor ($\beta$NGF), must be correctly formed in order to be active and suitable for use, and they must be free from any antigenic response. The process of obtaining the protein obtained from recombinant DNA could include glycosylation, the formation of correct disulphide bonds and other post-transductional modifications. The E.coli bacterial line cannot satisfy these requirements, while mammal eukaryotic cells and yeasts can. These are problems which must be considered when contemplating the potential application of human $\beta$NGF as a drug, obtained by biotechnology.

It has been demonstrated that NGF's activity depends on its dimeric form, that is, the assembly of two analogous polypeptides of 118 amino acids. Reduction with $\beta$ mercaptoethanol almost completely eliminates its biological activity. Attempts to renature the three disulphide bonds have resulted in a one-in-fifteen probability, from a statistical point of view, of the reassembly of the cysteines producing a correctly-structured molecule which is equal to the corresponding natural molecule. Consequently, when the molecule is obtained in E.coli there is no guarantee of the homology of its structure or its application as a drug to humans.

Indeed, once human NGF from E.coli has been purified to homogeneity, it shows, on immunoblotting with polyclonal antibodies specific to murine $\beta$NGF, a series of bands which cannot be attributed to the biologically active dimeric form. Moreover, the biological activity of this mixture of structures and forms has proved to be ten times greater than the analogous human form purified from natural sources such as placenta. On the one hand, this method of cloning and obtaining nerve growth factor in E.coli produces high levels of expression of the protein of interest. But, on the other, it produces a series of altered molecules which, when administered in vivo could give rise to secondary effects such as antibodies which would recognize and block the biological activity of the naturally present molecule. At the same time, cloning of the mature molecule in E.coli presents an initial methionine which cannot be removed and is certainly immunogenic, because it is placed in an exposed part of the molecule.

Another approach involves cloning prepro NGF in eukaryotic cells and consists in exploiting the attack of the specific peptidases naturally present in the eukaryotic cells to obtain the mature molecule. In particular, expression is effected on Spodoptera Frugiperda cells. The available literature shows that the whole genomic clone of human NGF has not yet been sequenced, but it has been demonstrated that the gene extends for over 10 kda (Ullrich, A. et al, Nature 303:821, 1983). Thus extended, the gene does not allow conventional cloning starting from this entire genomic sequence. The approach to be used is that of cloning a portion of the cDNA containing only the encoding portions of the protein. The complete cDNA for human βNGF has not yet been isolated (some sequences at 5' are still unknown) but much is now known about the NGF messengers of other origin (mouse, bovine, chick, etc) (Meier, R. et al, EMBO J 5:1489, 1986; Selby, H.J., J of Neuron Research 18:293, 1987) which could lead to some interesting deductions. The mouse NGF gene is present in a single copy and produces at least four different messengers of different dimensions (Selby, M.J. et al, Mol Cell Biol 7:3057, 1987). Their different dimensions are reflected above all in their different AUG initiator codons, the most important being at -187 and -121 with respect to the mature protein. These messengers are present in different quantities according to the different tissues. Those which begin at -187 are ten times more abundant in the submaxillary gland than those which begin at -121. However, assorted evidence has demonstrated that the highest percentage of NGF messengers expressed in the brain use these very AUG at -121.

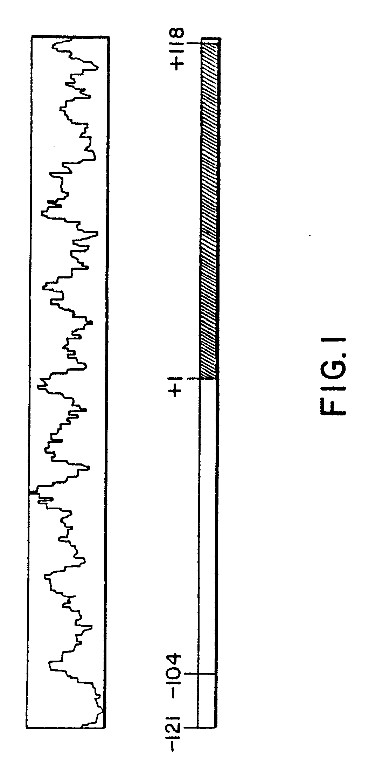

On the basis of this evidence, we cloned human βNGF starting from the methionine at -121. Analysis of the hydropathic profile showed that the amino acids between -121 and -104 can function as peptide leaders (Fig. 1), indicating that the protein can be secreted. To obtain the mature protein there is a specific peptidase with which it is possible to obtain the amino acid sequence from +1 to +118 corresponding to the biologically active peptide. This peptide is ubiquitous, since it is to be found in many types of mammal cell.Indeed, constructs containing the prepro NGF part, transfected into various types of cell are able to secrete mature NGF, allowing the production of a molecule of 14 kda in gel in denaturing and reducing conditions (Edwards, R.H., Mol Cell Biol 8:2456, 1988).

On the basis of the aforesaid previous experiences of obtaining the β subunit of nerve growth factor, we used a completely new approach to construct a vector to be used as a recombinant agent with the natural Baculovirus to infect Arthropoda Frugiperda cells. To clone the prepro NGF we used the PCR technique, with which we created restriction sites so as to keep the distances between the regulatory and encoding sequences as natural as possible and to allow easy cloning of the pVL vector. The portion of prepro NGF was joined to a sequence of modified, mature, human NGF (hβNGF), supplied by British Biotechnology Ltd (Oxford - Great Britain), in which restriction sites had been created to permit mutagenesis. For the purposes of the invention, the presence of these restriction sites must not be considered a limiting factor, and neither must the origin of the gene be considered so.

On this premise we cloned the prepro hβNGF under the functional control of the polyhedrin promoter of the Baculovirus. The terms "functional control" or "functionally linked" as used herein refer to a positioning of said prepro hβNGF relative to the polyhedrin promoter sequences which permits expression in the selected host cells, i.e. where the DNA nucleotides are present in a number which is neither too large nor too small for the gene construct itself to function, that is, it is precisely placed with no approximation. The construct was transfected into Spodoptera Frugiperda cells together with the natural Baculovirus. After purification a chimeric recombinant virus was isolated bearing in its genome or chromosome the human nerve growth factor (hNGF) encoding DNA sequence under the control of the Baculovirus polyhedrin promoter and of all the sequences delegated to its synthesis. This recombinant virus was used to reinfect the Arthropoda Frugiperda cells, demonstrating that the infected cells are able to produce in culture medium the polypeptide hβNGF in its mature and biologically active form. The prepro NGF portion proved to be essential to assembly of the molecule, allowing expression of the β subunit and demonstrating that the whole genetic construct was to be considered correct.

The present invention concerns a process for obtaining the β subunit of human NGF from a Baculovirus system, and more precisely:

- the preparation of a genetic construct such that the distance between the regulatory sequence (polyhedrin promoter) and the sequence encoding the protein of interest is natural;
- the agents which bring about recombination of the aforesaid construct with a wild-type virus; and
- the agents which allow the culturing and obtaining of the mature form of the β subunit of the human nerve growth factor (hNGF) in the absence of one or more amino acids fused to the polypeptide, different from those present in its natural sequence.

The polypeptide thus obtained shows biological activity when used on appropriate target cells. The hβNGF described in the present invention can be used to maintain nervous function or to prevent its loss, to recover it in chronic or acute pathological conditions, in neurodegenerative situations in the later stages of acute pathologies, such as cerebrovascular, infective, inflammatory, compressive, metabolic deficiencies, and in situations of modulation of the immune system. The polypeptide obtained is also free from other contaminating proteins of human origin, which could present undesired biological activity.

The invention is also directed towards the transformed cell line, which contains the vector in question, and its cultures which produce hβNGF.

Other objects of the present invention are pharmaceutical preparations which contain as active substances one or more of the new complexes between the β unit of the neuronotrophic factor and a natural ganglioside or one of its derivatives or semisynthetic analogues or one of its salts.

The invention also includes the therapeutic use of all the new complexes of gangliosides or their derivatives with the β subunit of the nerve growth factor NGF for the aforesaid indications. The following examples illustrate the preparation of the new compounds, process of their preparation, the pharmaceutical preparations and their uses.

The figures show:

Figure 1 is a hydropathic profile of the nerve growth factor polypeptide, β subunit, of human origin, according to the described method (Hopp et al, Proc Natl Acad Sci USA 78:3824, 1981). The position indicated as -121/-104 identifies the peptide leader while the position indicated as + 1/+118 indicates the amino acid sequence of the nerve growth factor polypeptide, β subunit, of human origin (Ullrich et al, Nature 303:821, 1983).

Figure 2 is a diagram of the construction of the expression vector pSVLhNGF.

Hereafter are detailed descriptions of how to accomplish the objects of the present invention. These descriptions are for illustrative purposes and do not limit the invention in any way and all variations which would appear evident to the expert in the art are to be considered as coming within its scope.

The attachments on the DNA chain with restriction enzymes were effected according to the manufacturer's instructions. Generally, 1 ug of plasmid was cut with 1 U of enzyme in 20 ul of solution; the temperature and incubation times depended on the enzyme used, generally 1 hour at 37°C. After incubation, the plasmids and gene segments were purified in all cases in an Agarose gel, LMP Agarose (BRL - United States of America) in 40 mM Tris HCl, 20 mM Sodium Acetate, 1 mM EDTA and then eluted from agarose with a GENECLEANTM kit (BIO 101 Inc, La Jolla, CA - USA). For the replication reactions at 5' the DNA was treated for 15 minutes at 15°C with 10 U of Polymerase (Klenow). To dephosphorize the DNA cut with restriction enzymes, bacterial alkaline phosphatase (GIBCO- -BRL) was used. The reaction was conducted in a 150 mM tris buffer pH 8 using 1 U of alkaline phosphatase in 20 ul at 60° for 1 hour. As ligase, the T4 ligase was used at a concentration of 1 U per 0.5 ug of DNA in a reaction of 20 ul at 13°C for 12 hours. Analyses to confirm the correct plasmid sequence were effected by transforming into HB101 cells and selecting the transforming cells into Agarose dishes in LB (Luria Bertani) medium with ampicillin antibiotic at 50 ug/ml. The plasmids contained in the HB101 were cultured in LB at 100 ug/ml of ampicillin and were then purified, both for the smaller and the larger preparations, with the kit by Quiagen (DIAGEN GmbH, Düsseldorf - Germany). The expression vectors were prepared from bacterial cells by the Quiagen method.

The DNA for the PCR reactions was prepared as follows from human placenta at term. A 0.4 cm3 piece of chorionic villi was finely chopped with scissors and suspended in 700 ul of 50 mM Tris/HCl pH 7.0, 100 mM EDTA, 100 mM NaCl, 1% SDS. To this was then added 35 ul of proteinase (K 100 ug/ml) and the whole was incubated overnight at 55°C. 20 ul of a 13 ug/ml solution of RNAase A were then added and it was incubated for another 2 hrs. This was followed by two extractions with phenol and two with chloroform. The DNA was then precipitated onto a fine glass tube by adding 1 volume of isopropanol. At this point it underwent several passages with 70% and 100% ethanol and was then dried. The DNA was then dissolved in buffer (10 mM Tris/HCl pH 7.4, 1 mM EDTA), in a test tube under gentle shaking. Some hours later, the dissolved DNA was ready for gene amplification. 0.1 ug of DNA was usually enough to proceed with PCR.

## Construction of the Transfer Vector

Since the Baculovirus genome is very ample (125 Kb) and has not yet been engineered with individual restriction sites, it is necessary to effect a recombination procedure between the transfer construct and the Baculovirus. In this way a chimeric Baculovirus is obtained in which the hNGF molecule has been engineered.

The transfer vector utilized was PVL (purchased from INVITROGEN, San Diego, CA), in which a single BamHI was created immediately after the polyhedrin. In order to clone in this vector, the hNGF molecule was engineered so as to add a splicing and poly-adenylation site immediately after the gene and the whole is contained between two BamHI sites to facilitate cloning.

Detailed Description of the Cloning of the Prepro NGF in the Transfer Vector for PVL Baculovirus

The prepro NGF was cloned by the PCR technique. Two oligonucleotides were synthesized: the first, between bases 9122 and 9147 (Ullrich, A., Nature 303:821, 1983), was to have the following sequence:

<div align="center">

**Met Ser Met Leu Phe**

**5'GCATAGCGTA ATG TCC ATG TTG TTC T3'**

</div>

The nucleotides before the initial AUG were changed and the synthesized oligonucleotide therefore has the following sequence:

<div align="center">

**XbaI     Met Ser Met Leu Phe**

**5'TGT CTAG AGT ATG TCC ATG TTG TTC T3'**

</div>

This oligonucleotide is called (XbaI). The second oligonucleotide, containing bases 9521 to 9342 (Ullrich, A., Nature 303:821, 1983), is complementary to this sequence to allow PCR and has the following sequence:

<div align="center">

**ECORI**

**5'GGCGG AATT CTCGGTGGTGGAC3'**

</div>

Within this oligonucleotide is contained the ECORI site to allow the union of the pre-pro NGF with the mature NGF. This oligonucleotide is called (ECORI). The two oligonucleotides were synthesized in solid phase on an oligonucleotide synthesizer by the method using phosphoramidites by the standard procedure for the 380B DNA Synthesizer (Applied Biosystems - USA). They were treated at 55°C for 12 hours in NH3 and then vacuum-dried. They were resuspended in 2.5 M ammonium acetate and then precipitated with 3 volumes of cold ethanol (-20°C). They were rewashed with 80% cold ethanol and resuspended in water. The concentration of the two oligonucleotides was assessed with a spectrophotometer. Amplification was carried out on a Perkin Elmer Cetus DNA Thermal Cycler and the reagents used for amplification were those contained in the relative DNATM AMplifier kit (Perkin Elmer-Cetus). A mixture of 200 uM of each oligonucleotide: 0.5 uM of each nucleotide dATP, dTTP, dCTP, dGTP and 0.1 ug of human DNA and reaction buffer in a total mixture of 100 ul with 0.5 U of TAQ polymerase, topped with paraffin oil to prevent evaporation. The amplification reaction was conducted by setting the instrument at 35 cycles in the case of human DNA. The cycle in both cases was as follows: 1 min at 94°C, 2 min at 45°C, 3 min at 72°C. The 300 bp amplified fragment was purified in a Low Melting Agarose gel (NuSieve) using the GENE-CLEANTM kit (BIO 101 Inc, La Jolla, CA - USA) to dissolve the Agarose. The DNA was cut with restriction enzymes XbaI and EcoRI and repurified as described above. The fragmented material thus purified was cloned in sites XbaI and EcoRI of the vector pGEM4 (Promega). The plasmid obtained was called pGEM4Xba-NGF. This plasmid was cut at HindIII-EcoRI and the fragment of 300 bp, once purified, was cloned in sites HindIII-EcoRI of the vector pUC18BBG26 (British Biotechnology Ltd, Oxford - UK). The plasmid pUC18BBG26 contains a synthetic hβNGF gene, which contains restriction sites not present in a natural gene, thereby allowing the substitution of certain domains, and the vector obtained is called pUC18hNGFC.

Subsequently this plasmid was cut at sites PvuII and XbaI and the fragment of 800 bp was cloned in the pGEM4 vector between sites PvuII and XbaI, thus obtaining the vector pGEMBamNGF.

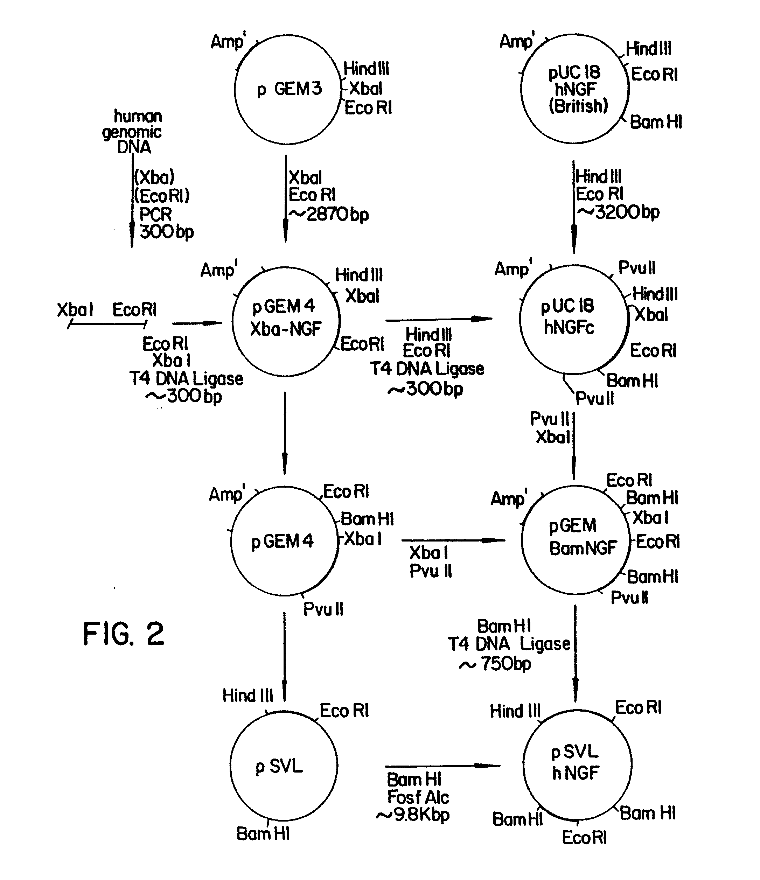

In this way the hNGF gene is included between two BamHI restriction sites. At this point the expression vector of the Baculovirus pVL was opened in BamHI dephosphorylated with alkaline phosphatase, after which the fragment of 760 bp BamHI of the pGEMBamNFG vector was inserted to obtain the expression vector pSVLhNGF. The different bacterial clones which bore the recombinant plasmid were analyzed to assess the correct position of the NGF gene with respect to the polyhedrin promoter. See Fig. 2 for a summary of the cloning.

## Insect Cell Cultures

The methods for the culture of these cells are per se well known to those skilled in this technology. Processes for their cultivation are described by Summers, H.D. et al. 1987, EP Publication No. 127 839, Smith G.E., and U.S. Patent No. 4,745,051.

In the present invention, cells of Spodoptera Frugiperda (Sf9) were used because they can be grown in monolayers or in suspensions. As monolayers they are maintained in the absence of CO2 at 25-30° and are divided 2-3 times a week at the confluent stage. Conditions for cultures in suspension depend on the medium and on the volume of the culture. About 2x106 cells/ml are grown in fermenters by high diffusion of air. Serum-free mediums are composed of Grace's insect medium culture (GIBCO BRL) as basic medium to which is added a lipid component and a peptone. An antibiotic is added as a protective agent. Pheronic F68 is used as protective agent against damage to cells from shaking.

## Preparation and Isolation of the Recombinant Virus

Detailed methods for the isolation of the recombinant virus can be found in the European Patent No. 0 127 839. In general, 2 ug of transfer vector DNA bearing the hNGF molecule and 1 ug of viral AcNPV DNA are cotransfected into a monolayer of Spodoptera Frugiperda cells. The cells present viral occlusions after 3-4 days and 10-50% of the cells are infected. The virus passes into the medium with a titer of 107 and of this 0.1%-0.5% are recombinant viruses. Various methods are known for the isolation of the recombinant virus. Hybridization plaques, with the hNGF gene as probe, or antibodies against m$\beta$NGF which cross-react with h$\beta$NGF can be used. In the case of the present invention, isolation of the recombinant virus was effected by serial dilutions in a monolayer of cells covered in soft agarose. After 2-3 cycles the recombinant virus presented negative occlusion characteristics. The plaques, containing about 10,000 viruses, were transferred into 2 ml of medium with a sterile pasteur pipette. Once isolated, this represents the recombinant virus which can then be used to infect and produce $\beta$hNGF in Spodoptera Frugiperda cells.

## Determination of the Biological Activity

In vitro studies to determine the biological activity of human NGF secreted in the culture medium of the Spodoptera Frugiperda cell line after infection with the recombinant virus were conducted in fetal pheochromocytoma PC-12 cells (Greene L.A. et al, A. Rev. Neurosci. 3:353, 1982). The specificity of the reaction was assessed using a culture medium of Spodoptera Frugiperda after infection with the natural virus or blocking the activity of the h$\beta$NGF present in the culture medium with polyclonal antibodies specific to $\beta$NGF of murine or bovine origin. The cells infected with the aforesaid recombinant virus produced the $\beta$ subunit of human NGF in a biologically active form and secreted in the culture medium. The proteins were assessed for expression in SDS gels of the supernatant.

## Pharmaceutical Compositions

Pharmaceutical compositions containing the human NGF molecule ($\beta$ subunit) derived from recombinant DNA, both with and without gangliosides and phospholipids, includes already known methods for the preparation of pharmaceutically acceptable compositions, which can be administered to patients and which allow an effective quantity of the hNGF molecule to be combined in a mixture with a pharmaceutically acceptable vehicle. Suitable vehicles and their formulations containing other proteins are described, for example in "Remington's Pharmaceutical Sciences" (Remingtons's Pharmaceutical Sciences. Mack Publishing Company, Easton, Pa., USA 1985). These vehicles include injectable "depot formulations". On this basis, the pharmaceutical formulation includes, albeit not exclusively, solutions of nerve growth factor or its freeze-dried powders in association with one or more pharmaceutically acceptable vehicles or diluents, and contained in buffered mediums with a suitable pH and isosmotic with the physiological fluids. In the case of freeze-dried preparations, it is possible to use supporting excipients, for example, but not exclusively, mannitol or glycine, and suitable buffered solutions of the desired volume will be supplied so as to obtain adequate isotonic buffered solutions with the desired pH. Similar solutions can be used for the pharmaceutical compositions of the nerve growth factor molecule obtained from recombinant DNA in isotonic solutions of the desired volume and include, but not exclusively, the use of buffered physiological solutions with phosphate or citrate at suitable concentrations so as to obtain each time isotonic pharmaceutical preparations of the desired pH, for example neutral pH.

Such pharmaceutical preparations may be for oral, rectal, parenteral, local, inhalatory, intracerebral uses. They are therefore in solid or semi-solid form, for example as pills, tablets, gelatin capsules, capsules, suppositories, soft gelatin capsules. For parenteral and intracerebral uses those forms for intramuscular or subcutaneous administration can be used, or those intended for infusion or intravenous or intracerebral injection, and they can therefore be in the form of solutions of the active compounds or as freeze-dried powders of the active compounds to be added to one or more pharmaceutically acceptable excipients or diluents, suitable for the aforesaid uses and with an osmolarity which is compatible with the physiological fluids. For local use, preparations in the form of creams or ointments for topical use should be considered; for inhalant uses, preparations in the form of sprays, for example nose sprays, should be considered. The preparations of the invention can be intended for administration to humans or animals. They contain preferably between 0.01% and 10% of active component in the case of solutions, sprays, ointments and creams and between 1% and 100% and preferably between 5% and 50% of active compound in the case of solid preparations.

Doses to be administered depend on individual needs, on the desired effect and on the chosen administration route. However, generally daily dosages to humans by injection (subcutaneous or intramuscular or intracerebral) vary from 0.05 mg to 5 mg of active substance per kg of body weight. The pharmaceutical formulations also include, but without being limited to the same, suppositories for rectal administration with lipophilic excipients, for example, hydroscluble, self-emulsifying excipients such as glycogelatin or others. In these preparations, the nerve growth factor obtained from recombinant DNA can be present in quantities varying between 0.01% and 1% in weight of the total excipient. The suppositories can contain, but are not limited to these, suitable quantities of acetylsalicylate.

For purely descriptive and not limiting purposes, we report hereafter some examples of pharmaceutical compositions prepared according to the present invention.

## A) EXAMPLES OF INJECTABLE SOLUTIONS

### Preparation No. 1 - one 2-ml vial contains:

| Active substance | µg | 1 (3,200 BU) |
|---|---|---|
| Sodium chloride | mg | 16 |
| Citrate buffer pH = 7 | ml | 2 |
| in water for injection | | |

### Preparation No. 2 - one 2-ml vial contains:

| Active substance | µg | 10 (32,000 BU) |
|---|---|---|
| Sodium chloride | mg | 16 |

Citrate buffer pH =7                           ml    2

in water for injection


Preparation No. 3 - one 2-ml vial contains:

Active substance                        µg    1 (3,200 BU)

Sodium chloride                         mg   16

Gangliosides as sodium salts            mg  100

Citrate buffer pH = 7                   ml    2

in water for injection


Preparation No. 4 - one 2-ml vial contains:

Active substance                        µg   10 (32,000 BU)

Sodium chloride                         mg   16

Gangliosides as sodium salts            mg   50

Citrate buffer pH = 7                   ml    2

in water for injection


Preparation No. 5 - one 2-ml vial contains:

Active substance                        µg    1 (3,200 BU)

Sodium chloride                         mg   16

Monosialotetrahexosylganglioside

(GM$_1$) sodium salts                   mg  100

Citrate buffer pH = 7                   ml    2

in water for injection


Preparation No. 6 - one 2-ml vial contains:

Active substance                        µg   10 (32,000 BU)

Sodium chloride                         mg   16

Monosialotetrahexosylganglioside

(GM$_1$) sodium salts                   mg  100

Citrate buffer pH = 7                   ml    2

in water for injection

Preparation No. 7

a)   one 2-ml ampoule contains:

| | | |
|---|---|---|
| Freeze-dried active substance | µg | 4 (12,800 BU) |
| Glycine | mg | 30 |

b)   one 2-ml ampoule of solvent contains:

| | | |
|---|---|---|
| Sodium chloride | mg | 16 |
| Citrate buffer in water | ml | 2 |
| Water for injection | | |


Preparation No. 8

a)   one 2-ml vial contains:

| | | |
|---|---|---|
| Freeze-dried active substance | µg | 4 (12,800 BU) |
| Mannitol | mg | 40 |

b)   one 2-ml ampoule of solvent contains:

| | | |
|---|---|---|
| Sodium chloride | mg | 16 |
| Citrate buffer in water | ml | 2 |
| Water for injection | | |


Preparation No. 9

a)   one 3-ml vial contains:

| | | |
|---|---|---|
| Freeze-dried active substance | µg | 10 (32,000 BU) |
| Glycine | mg | 45 |

b)   one 3-ml ampoule of solvent contains:

| | | |
|---|---|---|
| Sodium chloride | mg | 24 |
| Citrate buffer in water | ml | 3 |
| Water for injection | | |


Preparation No. 10

a)   one 3-ml vial contains:

| | | |
|---|---|---|
| Freeze-dried active substance | µg | 10 (32,000 BU) |
| Gangliosides as sodium salts | mg | 100 |
| Glycine | mg | 45 |

b)   one 3-ml ampoule of solvent contains:

| | | |
|---|---|---|
| Sodium chloride | mg | 24 |

Citrate buffer in water       ml  3

Water for injection

**Preparation No. 11**

a)   one 3-ml vial contains:

| | | |
|---|---|---|
| Freeze-dried active substance | µg | 10 (32,000 BU) |
| Gangliosides as sodium salts | mg | 50 |
| Glycine | mg | 45 |

b)   one 3-ml ampoule of solvent contains:

| | | |
|---|---|---|
| Sodium chloride | mg | 24 |
| Citrate buffer in water | ml | 3 |

Water for injection

**Preparation No. 12**

a)   one 3-ml vial contains:

| | | |
|---|---|---|
| Freeze-dried active substance | µg | 1 (3,200 BU) |
| Monosialotetrahexosylganglioside ($GM_1$) sodium salts | mg | 100 |
| Glycine | mg | 45 |

b)   one 3-ml ampoule of solvent contains:

| | | |
|---|---|---|
| Sodium chloride | mg | 24 |
| Citrate buffer in water | ml | 3 |

Water in injection

**Preparation No. 13**

a)   one 3-ml vial contains:

| | | |
|---|---|---|
| Freeze-dried active substance | µg | 10 (32,000 BU) |
| Monosialotetrahexosylganglioside ($GM_1$) sodium salts | mg | 100 |
| Glycine | mg | 45 |

b)   One 3-ml ampoule of solvent contains:

| | | |
|---|---|---|
| Sodium chloride | mg | 24 |
| Citrate buffer in water | ml | 3 |

Water for injection

Preparation No. 14

a)   one 3-ml vial contains:

| Freeze-dried active substance | µg | 10 (32,000 BU) |
| 3-sn-phosphatidyl)L-serine | mg | 50 |
| Lecithin | mg | 15 |
| Mannitol | mg | 100 |

b)   One 4-ml ampoule of solvent contains:

| Mannitol | mg | 60 |
| Water for injection to vol. | ml | 4 |


Preparation No. 15

a)   one 3-ml ampoule contains:

| Freeze-dried active substance | µg | 10 (32,000 BU) |
| Mannitol | mg | 60 |

b)   one 3-ml ampoule of solvent contains:

| Sodium chloride | mg | 24 |
| Citrate buffer in water | ml | 3 |
| Water for injection | | |


B)   EXAMPLES FOR SUBCUTANEOUS INJECTION


Preparation No. 16

a)   one 2-ml vial contains:

| Freeze-dried active substance | µg | 5 (16,000 BU) |
| Mannitol | mg | 30 |

b)   one 2-ml ampoule of solvent contains:

| Sodium chloride | mg | 16 |
| Citrate buffer in water | ml | 2 |
| Water for injection | | |

### C) EXAMPLES OF SUPPOSITORIES FOR THE RECTAL ROUTE

Preparation No. 17

| | | |
|---|---|---|
| Active substance | µg | 10 (32,000 BU) |
| Cocoa butter | mg | 2.5 |

Preparation No. 18

| | | |
|---|---|---|
| Active substance | µg | 10 (32,000 BU) |
| Carbowax 1540 | g | 1.75 |
| Carbowax 6000 | g | 0.75 |

Preparation No. 19

| | | |
|---|---|---|
| Active substance | µg | 10 (32,000 BU) |
| Tween 61 | g | 2.125 |
| Lanolin | g | 0.25 |

Preparation No. 20

| | | |
|---|---|---|
| Active substance | µg | 10 (32,000 BU) |
| Glycerine | g | 1.5 |
| Water | g | 0.25 |
| Gelatine | g | 0.25 |

The invention being thus described, it is clear that these methods can be modified in various ways. Such modifications are not to be considered as divergences from the spirit and purpose of the invention and any modification which would be apparent to an expert in the art comes within the scope of the following claims.

**Claims**

1. A recombinant Baculovirus wherein the transplacement genetic construct comprises:
   (a) a synthetic gene sequence including non-natural restriction sites and encoding the β subunit of the nerve growth factor polypeptide (βNGF) of human origin in its form known as mature or a biologically active derivative thereof; and wherein
   (b) the 5'-end of said gene sequence (a) is fused to the 3'-end of the natural sequence of the prepro region of human βNGF;
   (c) the 3'-end of said gene sequence (a) encoding human βNGF is fused to a polyadenylation and splicing sequence; and
   (d) the 5'- and 3'-end of said human prepro βNGF encoding DNA sequence present in said genetic construct is functionally linked to the regulatory elements of the polyhedrin promotor.

2. The recombinant Baculovirus according to claim 1 which is capable of expressing said human βNGF by cell infection in a culture medium.

3. The recombinant Baculovirus according to claim 1 or 2, wherein one or more amino acids fused to the amino acid sequence of the mature form known to date are absent from said genetic construct.

4. The recombinant Baculovirus according to claim 3, wherein said genetic construct contains the gene sequence encoding said mature form of the β subunit without said prepro region encoding DNA

14

sequence.

5.  A recombinant Baculovirus transplacement vector containing the genetic construct as characterized in any one of claims 1 to 4 and being capable of introducing it into the Baculovirus genome.

6.  A recombinant Baculovirus transplacement vector which is p SVLhNGF.

7.  A process for the preparation of a recombinant Baculovirus according to any one of claims 1 to 4, comprising introducing a recombinant Baculovirus transplacement vector according to claim 5 or 6 and a receptor Baculovirus into a host cell, allowing a transplacement of said genetic construct from said recombinant Baculovirus transplacement vector to said receptor Baculovirus, and isolating the recombinant Baculovirus of any one of claims 1 to 4.

8.  A recombinant insect cell containing a recombinant Baculovirus according to any one of claims 1 to 4 and being capable of expressing said human βNGF in its mature form.

9.  The recombinant insect cell according to claim 8 which is derived from Spodoptera Frugiperda.

10. A process for obtaining biologically active human nerve growth factor (βNGF), comprising culturing a recombinant cell according to claim 8 or 9 and recovering said human βNGF from the culture.

11. The process according to claim 10 for obtaining said β subunit in its mature form, wherein it is unnecessary to break away said β subunit from other subunits which may constitute its natural structure.

12. The process according to claim 10 or 11, wherein said human βNGF is obtained without a contamination by other products or proteins of human origin.

13. A biologically active human nerve growth factor (βNGF) obtained according to the process of any one of claims 10 to 12 and being free from other products or proteins of human origin.

14. A pharmaceutical preparation containing a biologically active human nerve growth factor (βNGF) according to claim 13.

15. The pharmaceutical preparation according to claim 14, additionally containing a natural ganglioside or one of its derivatives or semisynthetic analogues or one of its salts.

16. The pharmaceutical preparation according to claims 14 or 15 for administration to humans or animals.

17. The pharmaceutical preparation according to any of claims 14 to 16 which can be administered by parenteral route.

18. The pharmaceutical preparation according to any one of claims 14 to 16, which can be administered by intracerebral route.

19. The pharmaceutical preparation according to any one of claims 14 to 16, which can be administered by oral route.

20. The pharmaceutical preparation according to any one of claims 14 to 16, which can be administered by rectal route.

21. The pharmaceutical preparation according to any one of claims 14 to 16, which can be administered by local or topical route.

22. The pharmaceutical preparation according to any one of claims 14 to 16, which can be administered by inhalatory route.

23. The pharmaceutical preparation according to any one of claims 14 to 22 for the maintenance of nervous function.

24. The pharmaceutical preparation according to any one of claims 14 to 22 for the prevention of nervous function loss.

25. The pharmaceutical preparation according to any one of claims 14 to 22 for the recovery of nervous function in acute or chronic pathological conditions.

26. The pharmaceutical preparation according to claim 25, where the pathological conditions are acute, preferably cerebrovascular, infective, inflammatory, compressive or metabolic deficiencies.

27. The pharmaceutical preparation according to claim 25, where the pathological conditions are of chronic neurodegenerative type, including the late stages of acute pathologies.

28. The pharmaceutical preparation according to any one of claims 14 to 22 for the treatment of neuropathological conditions caused by aging of the nervous system or for the treatment of diseases affecting the immune system.

29. The pharmaceutical preparation according to any one of claims 14 to 22 for the modulation of the immune system.

FIG.1

FIG. 2